# EUROPÄISCHE PATENTANMELDUNG

(11) **EP 2 829 530 A1**
(43) Veröffentlichungstag der Anmeldung: **28.01.2015**
(21) Anmeldenummer: 13003758.3
(22) Anmeldetag: 27.07.2013
(51) Int. Cl.: C07C 37/045, C07C 51/367

(54) **Verfahren zur Herstellung von Phenolen**

(71) Anmelder: Weylchem Frankfurt Gmbh, 65933 Frankfurt Griesheim (DE)
(72) Erfinder: Forstinger, Klaus, 64832 Babenhausen (DE); Schwesinger, Reinhard, 79249 Merzhausen (DE); Maier, Andreas, 65817 Eppstein (DE)
(74) Vertreter: Wagner, Jutta

(57) **Zusammenfassung**

Die vorliegende Erfindung betrifft ein Verfahren zur Herstellung von Phenolen, bei dem ein Aryldiazoniumsalz, das durch die Diazotierung eines entsprechenden aromatischen, primären Amins hergestellt wird, durch Erhitzen in einem heißes Wasser, eine Mineralsäure und ein organisches Lösungsmittel enthaltenden Gemisch zersetzt wird, wobei das organische Lösungsmittel ein Keton der Formel (I) R¹C(O)R² 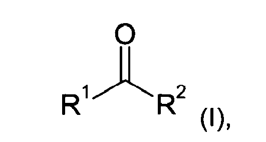 enthält, in der R¹ und R² unabhängig voneinander für (C₁-C₅)-Alkyl stehen und R¹ und R² zusammen mindestens vier Kohlenstoffatome aufweisen.

## Beschreibung

Die vorliegende Erfindung betrifft ein Verfahren zur Herstellung von Phenolen aus aromatischen Aminen.

Es ist bekannt, dass Phenole durch Verkochung von Diazoniumsalzen, welche durch die Diazotierung von aromatischen Aminoverbindungen mittels salpetriger Säure in der Kälte entstehen, hergestellt werden können. Gemäß "Ullmann's Encyclopedia of Industrial Chemistry" (7th Completely Revised Edition, vol. 11, p. 279) wird die Diazotierung und die Verkochung am besten in Schwefelsäure-Lösung durchgeführt. Es besteht jedoch die Problematik, dass bei der einfachsten Methode wie dem Erhitzen des Diazoniumsalzes auf bis zu 80-100 °C das erhaltene Produkt eine niedrige Reinheit aufweist. Eine Erhöhung der Ausbeute und Selektivität kann manchmal durch das Entfernen des gebildeten Phenols mittels aufwändiger, gleichzeitiger Dampfdestillation aus der Reaktionsmischung (s. z.B. DE 2 426 994 A1) oder durch das Lösen des entstehenden Phenols in Xylol als organisches Lösungsmittel erzielt werden.

Aus US 1910679 ist ein Verfahren zur Herstellung von Phenolen bekannt, bei welchem die Verkochung von entsprechenden Diazoniumsalzen in einer wässrigen sauren Lösung in Gegenwart eines organischen Lösungsmittels durchgeführt wird, wobei Anisol, Xylol oder Chlorbenzol als organische Lösungsmittel verwendet wurden. Jedoch ist die Ausbeute in diesem Verfahren nicht zufriedenstellend.

Die Aufgabe der Erfindung besteht daher darin, ein Verfahren zur Herstellung von Phenolen bereit zu stellen, durch das eine höhere Selektivität und Ausbeute von Phenolen bei deren Herstellung aus entsprechenden aromatischen primären Aminen durch die Diazoverkochung erzielt werden kann.

Überraschend wurde nun gefunden, dass eine Erhöhung der Selektivität und Ausbeute durch die Verwendung von aliphatischen Ketonen als Lösungsmittel bei der Diazoverkochung erzielt werden kann. Diese Ketone sind im Wesentlichen schlecht mit einem wässrigen Medium mischbar, in dem eine entsprechende Diazo-Verbindung einer Zersetzung unterzogen wird,.

Der Gegenstand der vorliegende Erfindung ist somit ein Verfahren zur Herstellung von Phenolen, bei dem ein Aryldiazoniumsalz, das durch die Diazotierung eines entsprechenden aromatischen, primären Amins hergestellt wird, durch Erhitzen in einem heißes Wasser und eine Mineralsäure sowie ein organisches Lösungsmittel enthaltenden Gemisch zum Phenol umgesetzt wird, wobei das organische Lösungsmittel ein Keton der Formel (I) R¹C(O)R² 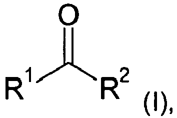 enthält, in der R¹ und R² unabhängig voneinander für (C₁-C₅)-Alkyl stehen und R¹ und R² zusammen mindestens vier Kohlenstoffatome aufweisen.

Im erfindungsgemäßen Verfahren erhält man überraschenderweise eine sehr hohe Selektivität im Vergleich zu Verfahren, bei denen entweder kein organisches Lösungsmittel oder Lösungsmittel wie Xylol, Chlorbenzol, Toluol, Methyl-*tert*-butylether (MTBE), Essigsäure-*n*-Butylester usw. verwendet werden. Außerdem kann durch das erfindungsgemäße Verfahren eine höhere Ausbeute erzielt werden.

In einer bevorzugten Ausführungsform des erfindungsgemäßen Verfahrens können R¹ und R² in der Verbindung der Formel (I) unabhängig voneinander folgende Bedeutung aufweisen: Methyl, Ethyl, *n*-Propyl, *iso*-Propyl, *n-*Butyl, *iso*-Butyl, *tert*-Butyl, *n*-Pentyl, *iso*-Pentyl und *tert-*Pentyl. Bevorzugt sind die Verbindungen, bei denen R¹ für Methyl oder Ethyl und R² für Ethyl oder *iso*-Butyl stehen. Insbesondere sind die Verbindungen der Formel (I) bevorzugt, in denen R¹ für Methyl und R² für *iso*-Butyl (Methylisobutylketon) oder R¹ und R² für Ethyl (3-Pentanon) stehen. Ganz besonders ist Methylisobutylketon als Verbindung der Formel (I) bevorzugt.

Die Diazotierungsreaktion wird in an sich bekannter Weise durchgeführt (s. z.B. "Ullmann's Encyclopedia of Industrial Chemistry", 7th Completely Revised Edition, vol. 11, p. 279). Das aromatische Amin wird üblicherweise in einem wässrigen Medium in Gegenwart einer Mineralsäure bei einer Temperatur von etwa -5 bis etwa 50 °C, vorzugsweise von etwa 0 bis etwa 20 °C, in Gegenwart eines zur Diazotierung geeigneten Reagenzes, bevorzugt in Gegenwart von salpeteriger Säure, diazotiert.

Die bevorzugten Amine umfassen Anilin und ein substituiertes Anilin, das mindestens einen weiteren Substituenten enthält, der ausgewählt ist aus: Alkyl, Alkenyl, Alkinyl, Halogen, Haloalkyl, Cycloalkyl, Heteroaryl, Carboxyl, Cyan, Alkoxyl und Ester. Bevorzugt werden folgende Gruppen: (C₁-C₆)-Alkyl, (C₂-C₆)-Alkenyl, (C₂-C₆)-Alkinyl, Fluor, Chlor, Brom, lod, (C₁-C₃)-Haloalkyl, (C₃-C₆)-Cycloalkyl, Heteroaryl, Carboxyl und Ester. Besonders bevorzugt sind primäre aromatische Amine, die Methyl-, Ethyl-, Fluor-, Chlor-, Brom-, lod-, CarboxylGruppen aufweisen.

Das Diazotiermittel wird im erfindungsgemäßen Verfahren im Allgemeinen in einer stöchiometrischen Menge oder einem kleinen Überschuss verwendet. Geeignet ist ein molares Verhältnis von Diazotiermittel zu aromatischem Amin von etwa 1 : 1 bis etwa 1,5 : 1, bevorzugt von etwa 1 : 1 bis etwa 1,1 : 1.

Als Diazotiermittel können z.B. Alkalinitrite, wie Natrium- oder Kaliumnitrit, Distickstofftrioxid und salpetrige Säure, Nitrosylhalogenide, wie Nitrosylchlorid oder -bromid, und andere Nitrosylsalze, wie Nitrosylperchlorat, - tetrafluorborat oder -sulfat verwendet werden. Natriumnitrit, Kaliumnitrit, Distickstofftrioxid oder salpetrige Säure werden bevorzugt, wobei Natriumnitrit besonders bevorzugt wird.

Die bei der Diazotierungsreaktion verwendete Mineralsäure hat vorzugsweise eine Konzentration von etwa 20 bis etwa 98 %. Besonders bevorzugt ist eine Konzentration von etwa 20 bis etwa 50 % oder von etwa 95 bis etwa 97 %. In einer bevorzugten Ausführungsform der Erfindung kann Schwefelsäure, Phosphorsäure, HCl oder deren Gemische als Mineralsäure bei der Diazotierungsreaktion verwendet werden. Schwefelsäure wird besonders bevorzugt.

Das gebildete Diazoniumsalz wird im Allgemeinen bei einer Temperatur von etwa 70 bis etwa 120 °C, vorzugsweise etwa 80 bis etwa 100 °C zersetzt (Verkochung). Vorzugsweise wird bei der Zersetzung das das Diazoniumsalz enthaltende Gemisch langsam einer Mischung aus einem Lösungsmittel der allgemeinen Formel (I), heißem Wasser und einer Mineralsäure zudosiert.

Typischerweise ist ein Mengenverhältnis des organischen Lösungsmittels zur wässrigen Mineralsäure von etwa 1:100 bis etwa 100:1, bevorzugt von etwa 1:25 bis etwa 25:1 geeignet. Die Konzentration der bei der Zersetzung verwendeten Mineralsäure kann im Bereich von etwa 10 bis etwa 98 %, bevorzugt etwa 20 bis etwa 96 %, liegen.

In einer bevorzugten Ausführungsform der Erfindung wird Schwefelsäure oder ein Gemisch aus Schwefelsäure und Phosphorsäure als Mineralsäure bei der Diazoverkochung verwendet. Schwefelsäure ist besonders bevorzugt.

Die Erfindung soll anhand der folgenden Beispiele erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

### Beispiel 1

In einem Rührkolben mit Thermometer, Rührer und Tropftrichter wurden 500 mL 24%ige Schwefelsäure vorgelegt. Dazu wurden 68 g Anthranilsäure gegeben. Diese Ausgangsmischung wurde bei 0°C bis 3 °C mit 87,8 g 40%iger Natriumnitrit-Lösung diazotiert. Die Diazoniumsalzlösung wurde dann bei 98°C und guter Rührung in eine Mischung aus 25 mL 24%iger Schwefelsäure und 500 mL Methyl-*iso-*butylketon eindosiert. Nach ca. 30 min. Nachreaktion bei 98°C wurde nach Abkühlung der Emulsion auf ca. 85°C eine Phasentrennung vorgenommen. Die organische Phase wurde mit einer Mischung aus 750 mL Wasser und 54 g 50%iger Natronlauge extrahiert. Nach erneuter Phasentrennung wurde aus der unteren wässerigen Phase das Zielprodukt (2-Hydroxybenzoesäure) durch Ansäuerung mit Salzsäure (pH<1,5) ausgefällt. Die Isolierung des Endproduktes erfolgte durch Filtration. Nach Waschen bis zum neutralen pH-Wert des Waschwassers und Trocknung des Filterkuchens wurden 64,1 g 2-Hydroxybenzoesäure mit einem Gehalt von 99,6% a/a (gemäß HPLC) erhalten. Das entspricht einer Ausbeute von: 93,5%. Die Selektivität betrug nach der Verkochung: 97,7% a/a (mittels HPLC bestimmt).

### Vergleichsbeispiel 2

Die Einsatzmengen und die Durchführung der Synthese waren die gleichen wie bei Beispiel 1. Als organisches Lösungsmittel wurde jedoch Xylol verwendet.
Auswaage Produkt: 55,8 g
Gehalt: 97,3% a/a (HPLC)
Ausbeute: 79,3%.
Selektivität nach Verkochung: 94,2% a/a (HPLC).

### Beispiel 3

In einem Rührkolben mit Thermometer, Rührer und Tropftrichter wurden 700 mL 22%ige Schwefelsäure vorgelegt. Dazu wurden 70,8 g 5-Chlor 2-methylanilin gegeben. Nach Heizen auf ca. 90°C und anschließender Abkühlung auf 10°C-12°C wurde die erhaltene Suspension mit 88,4 g 40%iger Natriumnitrit-Lösung diazotiert. Die Diazoniumsalzlösung wurde dann bei ca. 85-90°C und guter Rührung in eine Mischung aus 200 mL Methyl-*iso*-butylketon und 2 g 96%iger Schwefelsäure eindosiert. Nach ca. 30 min. Nachreaktion bei 85-90°C wurde nach Abkühlung der Emulsion auf ca. 70°C eine Phasentrennung vorgenommen. Die organische Phase wurde nicht weiter aufgearbeitet (destilliert). Es wurde die Selektivität mittels GC-Analyse ermittelt. Die Selektivität nach Verkochung betrug: 99,4% a/a (GC).

### Vergleichsbeispiel 4

Die Einsatzmengen und die Durchführung der Synthese waren die gleichen wie bei Beispiel 3. Als organisches Lösungsmittel wurde jedoch Xylol verwendet (Verkochungstemperatur: ca. 90°C). Die Selektivität nach Verkochung betrug: 94,1% a/a (GC).

### Beispiel 5

In einem Rührkolben mit Thermometer, Rührer und Tropftrichter wurden 700 mL 22%ige Schwefelsäure vorgelegt. Dazu wurden 70,8 g 3-Chlor-2-methylanilin gegeben. Nach Hochheizen auf ca. 90°C und anschließender Abkühlung auf 10°C-12°C wurde die vorliegende Suspension mit 88,4 g 40%iger Natriumnitrit-Lösung diazotiert. Die Diazoniumsalzmischung wurde dann bei ca. 90°C und guter Rührung in eine Mischung aus 200 mL Methyl-*iso*-butylketon und 2 g 96%iger Schwefelsäure eindosiert. Nach ca. 15 min. Nachreaktion bei 90°C wurde nach Abkühlung der Emulsion auf ca. 70°C eine Phasentrennung vorgenommen. Die organische Phase wurde noch einmal mit 50 mL Wasser gewaschen. Die Selektivität nach Verkochung betrug: 99,1% a/a (GC). Die organische Phase wurde dann destilliert. Es wurden 66,9 g 3-Chlor-2-methylphenol erhalten. Destillationsrückstand: 2,3 g. Der Gehalt des Zielproduktes betrug: 99,2% a/a (GC). Das entspricht einer Ausbeute von 93,1%.

### Beispiel 6

Die Einsatzmengen und die Durchführung der Synthese waren die gleichen wie bei Beispiel 5. Als organisches Lösungsmittel wurde jedoch 3-Pentanon verwendet. Die Selektivität nach Verkochung betrug: 96,9% a/a (GC).

### Vergleichsbeispiel 7

Die Einsatzmengen und die Durchführung der Synthese waren die gleichen wie bei Beispiel 5. Als organisches Lösungsmittel wurde jedoch Toluol verwendet. Die Selektivität nach Verkochung betrug: 83,0% a/a (GC). Endprodukt (destilliert): 58,7 g. Destillationsrückstand: 14,8 g. Der Gehalt des Zielproduktes betrug: 97,1% a/a (GC). Das entspricht einer Ausbeute von 80%.

### Beispiel 8

In einem Rührkolben mit Thermometer, Rührer und Tropftrichter wurden 375 mL 34%ige Schwefelsäure vorgelegt. Dazu wurden 60 g 3,4-Dimethylanilin gegeben. Nach Hochheizen auf ca.75°C und anschließender Abkühlung auf 0°C-3°C wurde die vorliegende Suspension mit 85,5 g 40%iger Natriumnitrit-Lösung diazotiert. Die Diazoniumsalzlösung wurde dann bei ca. 85°C und guter Rührung in eine Mischung aus 50 mL Methylisobutylketon und 120 mL 50%iger Schwefelsäure eindosiert. Nach ca. 30 min. Nachreaktion bei 85°C wurde, nach Abkühlung der Emulsion auf ca. 35°C, eine Phasentrennung vorgenommen. Die organische Phase wurde nicht weiter aufgearbeitet (destilliert). Es wurde die Selektivität mittels GC-Analyse ermittelt. Die Selektivität nach Verkochung betrug: 97,2% a/a (GC). Nach Abzug Lösemittel betrug die Ausbeute an Produkt 94,5 %.

### Vergleichsbeispiel 9

Die Einsatzmengen und die Durchführung der Synthese waren die gleichen wie bei Beispiel 8. Als organisches Lösungsmittel wurde jedoch Xylol verwendet. Die Selektivität nach Verkochung betrug: 93,1% a/a (GC). Nach Abzug Lösemittel betrug die Ausbeute an 3,4-Dimethylphenol 80,1 %.

### Vergleichsbeispiel 10

Die Einsatzmengen und die Durchführung der Synthese waren die gleichen wie bei Beispiel 8. Als organisches Lösungsmittel wurde jedoch Chlorbenzol verwendet. Die Selektivität nach Verkochung betrug: 94,2% a/a (GC). Nach Abzug Lösemittel betrug die Ausbeute an 3,4-Dimethylphenol 80,0 %

### Vergleichsbeispiel 11

Die Einsatzmengen und die Durchführung der Synthese waren die gleichen wie bei Beispiel 8. Bei der Verkochung wurde kein org. Lösungsmittel verwendet. Die Selektivität nach Verkochung betrug: 82,8% a/a (GC). Nach Abzug Lösemittel betrug die Ausbeute an 3,4-Dimethylphenol 59,2 %

### Vergleichsbeispiel 12

Die Einsatzmengen und die Durchführung der Synthese waren die gleichen wie bei Beispiel 8. Als organisches Lösungsmittel wurde jedoch Cyclohexanon verwendet. Die Selektivität nach Verkochung betrug: 80,2% a/a (GC). Nach Abzug Lösemittel betrug die Ausbeute an 3,4-Dimethylphenol 80,2 %.

### Vergleichsbeispiel 13

Die Einsatzmengen und die Durchführung der Synthese waren die gleichen wie bei Beispiel 8. Als organisches Lösungsmittel wurde jedoch Methylcyclohexan verwendet. Die Selektivität nach Verkochung betrug: 76,6 % a/a (GC). Nach Abzug Lösemittel betrug die Ausbeute an 3,4-Dimethylphenol 75,2%.

Wie die Ausführungsbeispiele zeigen, kann durch das erfindungsgemäße Verfahren eine deutliche Erhöhung der Selektivität und Ausbeute im Vergleich sowohl zu den bisher im Stand der Technik verwendeten organischen Lösungsmitteln wie Xylol oder Chlorbenzol als auch solchen Lösungsmitteln wie Toluol, Cyclohexanon oder Methylcyclohexan erzielt werden.

## Patentansprüche

1. Verfahren zur Herstellung von Phenolen, bei dem ein Aryldiazoniumsalz, das durch die Diazotierung eines entsprechenden aromatischen primären Amins hergestellt wird, durch Erhitzen in einem heißes Wasser, eine Mineralsäure und ein organisches Lösungsmittel enthaltenden Gemisch zersetzt wird,
**dadurch gekennzeichnet,**
**dass** das organische Lösungsmittel ein Keton der Formel (I) R¹C(O)R² 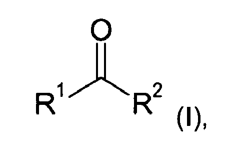 in der R¹ und R² unabhängig voneinander für (C₁-C₅)-Alkyl stehen und R¹ und R² zusammen mindestens vier Kohlenstoffatome aufweisen, enthält.

2. Verfahren nach Anspruch 1, **dadurch gekennzeichnet, dass** R¹ und R² unabhängig voneinander aus der Gruppe bestehend aus Methyl, Ethyl, *n-*Propyl, *iso*-Propyl, *n*-Butyl, *iso*-Butyl, *tert*-Butyl, *n*-Pentyl, *iso*-Pentyl, *tert-*Pentyl ausgewählt sind.

3. Verfahren nach Anspruch 1, **dadurch gekennzeichnet, dass** R¹ für Methyl oder Ethyl und R² für Ethyl oder *iso*-Butyl stehen, vorzugsweise R¹ für Methyl und R² für *iso*-Butyl oder R¹ und R² für Ethyl stehen.

4. Verfahren nach Anspruch 1, **dadurch gekennzeichnet, dass** Methylisobutylketon oder 3-Pentanol, bevorzugt Methylisobutylketon, als Keton der Formel (I) verwendet wird.

5. Verfahren nach einem oder mehreren der Ansprüche 1-4, **dadurch gekennzeichnet, dass** die Konzentration der Mineralsäure von etwa 10 bis etwa 98 %, bevorzugt von etwa 20 bis etwa 50 % oder von etwa 95 bis etwa 97%, beträgt.

6. Verfahren nach einem oder mehreren der Ansprüche 1-5, **dadurch gekennzeichnet, dass** das Mengenverhältnis von organischem Lösungsmittel der Formel (I) zur wässrigen Lösung der Mineralsäure etwa 1:100 bis etwa 100:1, bevorzugt etwa 1:25 bis etwa 25:1 beträgt.

7. Verfahren nach einem oder mehreren der Ansprüche 1-6, **dadurch gekennzeichnet, dass** Schwefelsäure oder Phosphorsäure, bevorzugt Schwefelsäure, als Mineralsäure eingesetzt wird.

8. Verfahren nach einem oder mehreren der Ansprüche 1-8, **dadurch gekennzeichnet, dass** das Aryldiazoniumsalz bei einer Temperatur von etwa 70 bis etwa 120 °C, vorzugsweise von etwa 80 bis etwa 110 °C zersetzt wird.
